Europäisches Patentamt

European Patent Office

Office européen des brevets

(11) Publication number: 0 375 213
A1

## (12) EUROPEAN PATENT APPLICATION

(21) Application number: 89312772.0

(22) Date of filing: 07.12.89

(51) Int. Cl.5: C12P 1/00, C12P 21/00, A61K 37/00, //(C12P1/00, C12R1:01)

The applicant has filed a statement in accordance with Rule 28 (4) EPC (issue of a sample only to an expert). Accession number(s) of the deposit(s): NCIB 40086.

Claims for the following Contracting State: ES.

The title of the invention has been amended (Guidelines for Examination in the EPO, A-III, 7.3).

(30) Priority: 07.12.88 GB 8828513

(43) Date of publication of application:
27.06.90 Bulletin 90/26

(84) Designated Contracting States:
ES GR

(71) Applicant: BEECHAM GROUP PLC
Beecham House Great West Road
Brentford Middlesex TW8 9BD(GB)

(72) Inventor: Coates, Nigel John
Beecham Pharmaceuticals Research Division
Brockham Park Betchworth Surrey RH3 7AJ(GB)
Inventor: Elson, Anna Louisa
Beecham Pharmaceuticals Research Division
Brockham Park Betchworth Surrey RH3 7AJ(GB)
Inventor: Athalye, Medha
Beecham Pharmaceuticals Research Division
Brockham Park Betchworth Surrey RH3 7AJ(GB)
Inventor: Curtis, Lawrence Mary
Beecham Pharmaceuticals Research Division
Brockham Park Betchworth Surrey RH3 7AJ(GB)
Inventor: Moores, Linda Vivienne
Faraway Cottage Trap Lane Walliswood
Dorking Surrey RH5 5QX(GB)

(74) Representative: West, Vivien et al
Beecham Pharmaceuticals Great Burgh Yew Tree Bottom Road
Epsom Surrey KT18 5XQ(GB)

(54) Complex of glycopeptide compounds MM 55270, MM 55271 or MM 55272.

(57) New glycopeptide antibiotics MM55270, MM55271, and MM55272 are produced by fermentation of Amycolatopsis sp. NCIB 40086.

## NOVEL PRODUCTS

The present invention relates to novel antibacterially active materials obtainable from a microorganism, to processes for their production, and to their pharmaceutical use.

A large number of microorganisms have been isolated from nature and certain of those microorganisms have been found to produce various metabolites, which can be isolated and some of which have useful antibacterial activity. One such metabolite is a substance which is believed to be a novel complex of glycopeptide compounds and has been found to have useful antibacterial activity.

The present invention accordingly provides the novel complex of glycopeptide compounds which is obtainable as described hereinbelow in Example 1 or 2.

The complex has the following characteristics:

(i) it may be obtained by the cultivation of a microorganism of the order Amycolatopsis;

(ii) the complex comprises at least nine glycopeptide compounds, including three compounds which have been designated MM55270, MM55271 and MM55272; and

(iii) it shows antibacterial activity against Staphylococcus aureus V573.

The said MM55270, MM55271 and MM55272 are believed to be novel glycopeptide compounds, and each therefore forms a further aspect of the invention, together with its production and use. They have the characterising data set out hereinbelow in the experimental section.

The present invention also provides a process for the production of a complex or compound in accordance with the invention which comprises cultivating a producing microorganism and subsequently isolating the product or a derivative thereof from the culture.

The present invention furthermore provides a process for the preparation of a complex or compound in accordance with the invention which comprises separating the product or a derivative thereof from a solution thereof in admixture with other antibacterially active substances and/or inactive substances by adsorption onto an affinity resin.

The term 'cultivation' (and derivatives of that term) as used herein means the deliberate aerobic growth of an organism in the presence of assimilable sources of carbon, nitrogen, sulphur and mineral salts. Such aerobic growth may take place in a solid or semi-solid nutritive medium, or in a liquid medium in which the nutrients are dissolved or suspended. The cultivation may take place on an aerobic surface or by submerged culture. The nutritive medium may be composed of complex nutrients or may be chemically defined.

It has been found that suitable microorganisms for use in the cultivation process according to the invention include bacterial strains belonging to the order Amycolaptopsis. It has further been found that an example of such a strain is sp. NCIB 40086 and also mutants thereof, which has been isolated from nature.

The term 'mutant' as used herein includes any mutant strain which arises spontaneously or through the effect of an external agent whether that agent is applied deliberately or otherwise. Suitable methods of producing mutant strains including those outlined by H.I. Adler in 'Techniques for the Development of Microorganisms' in 'Radiation and Radioisotopes for Industrial Microorganisms', Proceedings of a Symposium, Vienna, 1973, page 241, International Atomic Energy Authority, and these include:

(i) Ionizing radiation (e.g. X-rays and λ-rays), u.v. light, u.v. light plus a photosensitizing agents (e.g. 8-methoxypsoralen), nitrous acid, hydroxylamine, pyrimidine base analogues (e.g. 5-bromouracil), acridines, alkylating agents (e.g. mustard gas, ethyl-methane sulphonate), hydrogen peroxide, phenols, formaldehyde, heat, and

(ii) Genetic techniques, including, for example, recombination, transformation, transduction, lysogenisation, lysogenic conversion, protoplast fusion and selective techniques for spontaneous mutants.

Sp. NCIB 40086 is believed to be a previously unreported species in the order Amycolatopsis and therefore also forms a part of the present invention, particularly in biologically pure form. It has been deposited at the National Collections of Industrial and Marine Bacteria Ltd. (N.C.I.B), Aberdeen, Scotland under number 40086 on 28th November, 1988.

The fermentation medium for cultivating sp. NCIB 40086 suitably contains sources of assimilable carbon and assimilable nitrogen together with inorganic salts. Suitable sources of nitrogen include yeast extract, soyabean flour, meat extract, cottonseed, flour, malt, distillers dried solubles, amino acids, protein hydrolysates and ammonium and nitrate nitrogen. Suitable carbon sources include glucose, lactose, maltose, starch and glycerol. Suitably the culture medium also includes alkali metal ions (for example, sodium), halogen ions (for example, chloride), and alkaline earth metal ions (for example calcium and magnesium), as well as trace elements such as iron and cobalt.

The cultivation may suitably be effected at a temperature of about 20 to 35°C , advantageously 20 to

30°C, and the culture may suitably be harvested up to 7 days, advantageously about 3 to 5 days, after the initiation of fermentation in order to give an optimum yield.

The desired product or a derivative thereof may then be isolated from the culture medium and worked up and purified using conventional techniques for glycopeptide compounds. All such isolation and purification procedures may conveniently be effected at cool to ambient temperature, for example at a temperature within the range of from 4 to 30°C, conveniently from 20 to 25°C.

The desired product is generally obtained predominantly from the culture filtrate, and it is therefore convenient for the first isolation step to involve removal of solid material from the fermentation broth by, for example, filtration or centrifugation, to give a clarified culture filtrate.

Further isolation of the desired product from the clarified culture filtrate may conveniently be effected by adsorption onto an affinity resin such as D-alanyl-D-alanine-sepharose affinity resin.

The desired product may readily be identified in a routine manner by testing for antibacterial activity and/or by monitoring the h.p.l.c. retention time.

Suitably, the separation procedure may include a high-performance liquid chromatography step, preferably as the last step. Elution may be effected using aqueous $NaH_2PO_4$/acetonitrile.

Each product according to the invention is suitably provided in substantially pure form, for example at least 50% pure, suitable at least 60% pure, advantageously at least 75% pure, preferably at least 85% pure, more preferably at least 95% pure, especially at least 98% pure, all percentages being calculated as weight/weight. An impure or less pure form of a product according to the invention may, for example, be used in the preparation of a more pure form of the same product or of a related product (for example a corresponding derivative) suitable for pharmaceutical use.

The products of the invention have antibacterial properties and are useful for the treatment of bacterial infections in animals, especially mammals, including humans, in particular humans and domesticated animals (including farm animals). They may be used for the treatment of infections caused by a wide range of organisms including, for example, those mentioned herein.

The present invention provides a pharmaceutical composition comprising a product according to the invention or a pharmaceutically acceptable derivative thereof together with a pharmaceutically acceptable carrier or excipient.

The present invention also provides a method of treating bacterial infections in animals, especially in humans and in domesticated mammals, which comprises administering a product according to the invention or a pharmaceutically acceptable derivative thereof, or a composition according to the invention, to a patient in need thereof.

The products and compositions according to the invention may be formulated for administration in any convenient way for use in human or veterinary medicine, by analogy with other antibiotics.

The products and compositions according to the invention may be formulated for administration by any route, for example oral, topical or parenteral. The compositions may, for example, be made up in the form of tablets, capsules, powders, granules, lozenges, creams, syrups, or liquid preparations, for example solutions or suspensions, which may be formulated for oral use or in sterile form for parenteral administration by injection or infusion.

Tablets and capsules for oral administration may be in unit dosage form, and may contain conventional excipients including, for example, binding agents, for example, syrup, acacia, gelatin, sorbitol, tragacanth, or polyvinylpyrrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; and pharmaceutically acceptable wetting agents, for example sodium lauryl sulphate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or another suitable vehicle before use. Such liquid preparations may contain conventional additives, including, for example, suspending agents, for example sorbitol, methyl cellulose, glucose syrup, gelatin, hydroxyethyl cellulose, carboxymethyl cellulose, aluminium stearate gel or hydrogenated edible fats; emulsifying agents, for example lecithin, sorbitan monooleate or acacia; non-aqueous vehicles (which may include edible oils), for example almond oil, oily esters (for example glycerine), propylene glycol, or ethyl alcohol; preservatives, for example methyl or propyl p-hydroxybenzoate or sorbic acid; and, if desired, conventional flavouring and colour agents.

Compositions according to the invention intended for topical administration may, for example, be in the form of ointments, creams, lotions, eye ointments, eye drops, ear drops, impregnated dressings, and aerosols, and may contain appropriate conventional additives, including, for example, preservatives, solvents to assist drug penetration, and emollients in ointments and creams. Such topical formulations may also contain compatible conventional carriers, for example cream or ointment bases, and ethanol or oleyl alcohol for lotions. Such carriers may constitute from about 1% to about 98% by weight of the formulation; more usually they will constitute up to about 80% by weight of the formulation.

Compositions according to the invention may be formulated as suppositories, which may contain conventional suppository bases, for example cocoa-butter or other glycerides.

Compositions according to the invention intended for parenteral administration may conveniently be in fluid unit dosage forms, which may be prepared utilizing the compound and a sterile vehicle, water being preferred. The active ingredient(s) depending on the vehicle and concentration used, may be either suspended or dissolved in the vehicle. In preparing solutions, the product may be dissolved in water for injection and filter-sterilized before being filled into a suitable vial or ampoule, which is then sealed. Advantageously, conventional additives including, for example, local anaesthetics, preservatives, and buffering agents can be dissolved in the vehicle. In order to enhance the stability of the solution, the composition may be frozen after being filled into the vial, and the water removed under vacuum; the resulting dry lyophilized powder may then be sealed in the vial and a accompanying vial of water for injection may be supplied to reconstitute the liquid prior to use. Parenteral suspensions may be prepared in substantially the same manner except that the product is suspended in the vehicle instead of being dissolved and sterilisation cannot be accomplished by filtration. The product may instead be sterilised by exposure to ethylene oxide before being suspended in the sterile vehicle. Advantageously, a surfactant or wetting agent is included in such suspensions in order to facilitate uniform distribution of the product.

A product or composition according to the invention may suitable be administered to the patient in an antibacterially effective amount.

A composition according to the invention may suitably contain from 0.1% by weight, preferably from 10 to 60% by weight, of a product according to the invention (based on the total weight of the composition), depending on the method of administration.

The products according to the invention may suitably be administered to the patient at a daily dosage of from 1.0 to 50 mg/kg of body weight. For an adult human (of approximately 70 kg body weight), from 50 to 3000 mg, for example about 1500 mg, of a product according to the invention may be administered daily. Suitably, the dosage for adult humans is from 5 to 20 mg/kg per day. Higher or lower dosages may, however, be used in accordance with normal clinical practice.

When the compositions according to the invention are presented in unit dosage form, each unit dose may suitably comprise from 25 to 1000 mg, preferable from 50 to 500 mg, of a product according to the invention.

The following Examples illustrate the preparation of products according to the present invention.

## Production and Isolation of Glycopeptide complex Example 1.

### a) Fermentation.

Culture NCIB 40086 was grown for 7 days at 26°C on a solid agar slant in a McCartney bottle. The agar medium had the following composition:

| Constituent | Amount (g/l) |
|---|---|
| Yeast extract | 4.0 |
| Malt extract | 10.0 |
| Dextrose | 4.0 |
| Agar | 20.0 |
| Deionised water | To 1 litre |

[The constituents were all 'Bacto' products (Bacto is a Trade Mark) as supplied by Difco Laboratories, P.O. Box 14B, Central Avenue, East Molesey, Surrey].

The medium was adjusted to pH 7.3 before sterilization.

A spore suspension was prepared by adding 10ml of sterilized water containing 0.005% Triton X 100 to a McCartney bottle agar culture of NCIB 40086, followed by sonication of the suspension for 1 minute. [Triton X 100 was obtained from B.D.H Chemicals Ltd., Poole, Dorset]. Portions (1ml) of spore suspension were used to inoculate the fermentation medium (100ml) contained in 500ml conical flasks closed with foam plastic plugs. The fermentation medium contained:

| Constituent | Amount (g/l) |
|---|---|
| Soya bean flour | 10 |
| Glycerol | 20 |
| Maltose | 2 |
| $CoCl_2.6H_2O$ | 0.005 |
| Trace element solution | 10ml |
| Deionised water | to 1 litre |

The stock trace element solution contained:

| Constituent | Amount |
|---|---|
| $CaCl_2.2H_2O$ | 10 |
| $MgCl_2.6H_2O$ | 10 |
| NaCl | 10 |
| $FeCl_3.$ | 3 |
| $ZnCl_2$ | 0.5 |
| $CuCl_2.2H_2O$ | 0.5 |
| $MnSO_4.4H_2O$ | 0.5 |

The medium was adjusted to pH 7.3 before sterilization at 117°C for 15 minutes.

[The soya bean flour was Arkasoy 50 supplied by Arkady - A.D.M., Manchester, U.K.].

Incubation was carried out for 120hrs at 26°C and 240r.p.m. on a gyratory shaker. The harvested broth was clarified by centrifugation. Samples were monitored for antibiotic activity by bioassay on Staphylococcus aureus using the conventional hole-in-plate method.

b) Isolation of complex

The clarified broth (1500ml) prepared as described in a) was stirred for 1 hour with D-alanyl-D-alanine-sepharose affinity resin (15ml wet volume). The affinity adsorbent was prepared from D-alanyl-D-alanine immobilised on activated CH-Sepharose 4B [6-Aminohexanoic acid-activated-sepharose-4B was obtained from Sigma Chemical Co., Poole, Dorset].

The mixture was filtered onto a glass scinter funnel and the filtrate discarded. The affinity resin was resuspended in deionised water (750ml) and filtered as before. The resin was washed once more with deionised water. The glycopeptide complex was eluted from the affinity resin with 800ml of 0.1M ammonia containing 50% acetonitrile. The eluate was evaporated under reduced pressure and freeze-dried to yield 47mg of the complex.

The complex (81mg) prepared essentially as above was dissolved in 500ml of 0.05M $NaH_2PO_4$ pH 6.5. This solution was then applied to a 260ml CM Sephadex C25 cation exchange column (Na$^+$ form) previously equilibrated with 0.05M $NaH_2PO_4$ pH 6.5. (CM Sephadex was supplied by Pharmacia Ltd., Uppsala, Sweden). The column was washed with 0.05M $NaH_2PO_4$ pH 7.0 (750ml), and then eluted using an exponential gradient of 0.05M $NaH_2PO_4$ pH 7.0 to 0.2M $Na_2HPO_4$ pH 9.4 (500ml in mixing vessel). 16ml fractions were collected. Antibacterial activity was detected in the phosphate washes and in fractions 1-10.

Inorganic impurities were removed from the separate bulks by absorbing once more onto D-alanyl-D-alanine sepharose affinity resin. The resin was washed with deionised water (3 x 200ml aliquots) and the glycopeptides eluted with 0.1M ammonia containing 50% acetonitrile (200ml). The percolate and water washes were discarded. The ammonia and acetonitrile from each bulk were evaporated off in vacuo and the samples freeze-dried. 9.3mg of the complex was obtained from the phosphate wash bulk, whilst 2.0mg was obtained from the fraction 1-10 bulk. The samples were monitored by high performance liquid chromatography using a 3.9 x 300mm Waters μbondapak $C_{18}$ reverse phase column, eluting with 0.1M $NaH_2PO_4$ pH

6.0 containing 11% acetonitrile at a flow rate of 2ml/minute. The eluate was monitored for UV absorbance at 220nm. Both samples were composed of the same complex of nine glycopeptides. The former containing a major component with a retention time of 8.8 minutes, the latter at 28.8 minutes (Vancomycin had a retention time of 5.8 minutes in this system).

FAB mass spectroscopy indicated a molecular ion (MH+) of 1489±1 for the component with a retention time of 8.8 minutes designated MM55270 and 1517±1 for that at 28.8 minutes designated MM55271.

## Reference Example

### Preparation of affinity adsorbent

The N-hydroxysuccinimide ester of 6-aminohexanoic acid Sepharose 4B (60g) was placed on a glass scinter and washed with 1mM hydrochloric acid solution (2L) under suction. The wet cake was then added to a solution of D-alanyl-D-alanine (1.5g) in 0.1M sodium bicarbonate solution (60ml) and occasionally shaken over the next hour. The suspension was filtered under suction and the residue suspended in 0.1M tris (hydroxymethyl)aminomethane (TRIS) (100ml) for 1 hour and then refiltered through a glass sinter. The cake was washed successively with 0.1M sodium bicarbonate solution, 0.05M TRIS (containing 0.5M sodium chloride), 0.05M formate buffer at pH 4.0 (containing 0.5M sodium chloride) and finally distilled water. The affinity resin was then stored at 4°C in aqueous suspension.

## Example 2

### a) Fermentation

All seed and production stages used the liquid medium as described in Example 1a. A 1ml vegetative cell suspension of culture NC1B 40086 stored in 20% glycerol and 10% lactose under liquid nitrogen, was used to inoculate 100ml of fermentation medium contained in a 500ml conical flask, stoppered with a foam plastic bung. After incubation for 72 hours at 28°C and 240 rpm on a gyratory shaker, 15ml cell culture was transferred to 500ml fresh medium in a 2 litre conical flask. Incubation was again maintained for 72 hours at 28°C and 240rpm on a gyratory shaker.

15 litres of fermentation medium together with 0.1% antifoaming agent polypropylene glycol P2000 was sterilized for 60 minutes at 121°C in a 20 litre fully baffled fermenter. The fermenter was stirred by a bottom drive agitator fitted with three vaned-disc impellers at 200rpm during sterilization and 300rpm during cultivation. 500ml vegetative inoculum from the second stage seed flask was used to inoculate the fermenter and incubation wass carried out at 28°C for 95 hours before transfer to the final stage.

During the fermentation, the fermenter was supplied with sterile filtered air at 0.23 volumes per volume per minute and an overpressure of air of 0.5 bar was maintained throughout.

300 litres of fermentation medium together with 0.1% antifoaming agent polypropylene glycol P2000 was sterilized for 60 minutes at 121°C in a 450 litre fully baffled fermenter. During sterilisation the fermenter was stirred at 100rpm by a bottom driven agitator fitted with three vaned-disc impellers. 15 litres of vegetative culture from the 20 litre fermenter was used to inoculate the 450 litre fermenter.

Incubation was carried out at 28°C for 78 hours before harvest. During the fermentation, the fermenter was stirred at 50rpm and supplied with sterile filtered air at 0.5 volumes per volume per minute. An overpressure of air of 0.5 bar was maintained throughout.

### b) Isolation of the complex

The neutralised clarified broth (350 L) was passed through a 21L IRA 458 anion exchange column (Cl⁻ form) at a flow rate of 1 L.min⁻¹. [Resin supplied by Rohm and Haas Ltd., Lenning House, 2 Mason's Avenue, Croydon, Surrey. England]. The partly decolourised percolate containing the antibiotic activity, was applied to a 22.6L column of Diaion HP20 at a flow rate of 1L min⁻¹. [Resin supplied by Mitsubishi Chemical Industries, Tokyo, Japan.] The column was washed with 10L of deionised water and the percolate and water wash discarded. The active material was eluted from the column with 0.1M ammonia containing 50% propan-2-ol. 1 litre fractions were collected. Fractions with antibiotic activity, (65-96), were bulked and evaporated in vacuo to 10L.

The aqueous concentrate was filtered through GF/D glass fibre filters. [Filters supplied by Whatman, Springfield Mill, Maidstone, Kent, England.]

The filtrate was stirred for 1 hour with D-alanyl-D-alanine-sepharose affinity resin, (350 ml wet volume), [Prepared as in example 1b]. The mixture was treated as previously described and the combined eluates were evaporated in vacuo and freeze dried to yield 4.9g of the complex.

Example 3

a) Separation of glycopeptides MM 55271 and MM 55272 from the glycopeptide complex.

194mg of glycopeptide complex prepared essentially as described in Example 2, was dissolved in 100ml of 0.05M $NaH_2PO_4$ buffer pH 5.0, and loaded onto a 300ml SP Sephadex C25 cation exchange column ($Na^+$ form), previously equilibrated with the same buffer. [SP Sephadex was supplied by Pharmacia, Uppsala, Sweden.] The column was washed with 0.05M $NaH_2PO_4$ buffer pH 5.0 (300ml) and then eluted with an exponential gradient of 0.05M $NaH_2PO_4$ pH 5.0 to 0.2M $Na_2HPO_4$ pH10.5 (250ml in the mixing vessel), at a flow rate of 2.5ml/ml. 10ml fractions were collected. Fractions were monitored by bioassay on Staphylococcus aureus V573.

The SP Sephadex percolate, buffer wash and eluate fractions 18-23 were antibacterially active. The percolate and buffer wash were combined. Eluate fractions 18-23 were bulked separately. The bulked fractions were desalted with D-alanyl-D-alanine sepharose affinity resin (10ml wet volume) as described in Example 1. The SP Sephadex percolate and wash yielded 69mg of white solid, and 43mg was obtained from the SP Sephadex eluate fractions 18-23. The products were assayed by HPLC on a Spherisorb column (250 x 4.6mm) containing 10 micron C18 Spherisorb ODS2 reverse phase material, [supplied by Phase Separation Ltd., Deeside Industrial Park, Queensferry, Clwyd, UK]. The HPLC mobile phase was 0.1M $NaH_2PO_4$ pH 6.0 containing 12% acetonitrite, flow rate 2ml/min, monitored at 210nm.

HPLC showed that both products contained a major glycopeptide at a retention time of 9.0 minutes. The glycopeptide isolated from the SP Sephadex percolate and buffer wash was designated MM 55271 and the glycopeptide isolated from the SP Sephadex eluate fractions was designated MM 55272.

b) Further purification of MM 55271

The 69 mg of solid containing MM 55271 was taken up in 0.1M $NaH_2PO_4$ buffer pH 6.0 (25ml) and loaded onto a 22.5ml column containing Matrex silica C18 chromatography medium equilibrated in the same buffer. [Matrex silica C18 was supplied by Amicon Corp, Danvers, MA 01923 USA]. The column was eluted with an exponential gradient of 0.1M $NaH_2PO_4$ pH6.0, to 0.1M $Na H_2PO_4$ pH 6.0 containing 20% acetonitrile (200ml in mixing vessel). 10ml fractions were collected. The fractions were monitored for biological activity and by HPLC. Fractions 20 - 24 were substantially pure MM 55271. The fractions were bulked and the acetonitrile evaporated off in vacuo. The buffer salts were removed by treatment with D-alanyl-D-alanine affinity resin as previously described to yield 17.4mg of MM 55271. Fast atom bombardment mass spectroscopy indicated a molecular weight of 1516±1.

The antibacterial activity of MM 55271 against a range of Gram positive organisms is shown in the following table.

| Antibacterial activity of MM 55271 against a range of organisms, determined by the microtitre method | |
| --- | --- |
| (MIC µg/ml) | |
| Organisms | MM 55271 |
| Bacillus subtilis ATCC 6633 | 8 |
| Coynebacterium xerosis NCTC 9755 | 4 |
| Sarcina lutea NCTC 8340 | 16 |
| Staphylococcus aureus | |
| 'Oxford' | 8 |
| 'Russell' | 16 |
| 'V573' MR* | 4 |
| S.saprophyticus 'FLI' | 16 |
| S.epidermidis 60137 | 8 |
| 54815 | 32 |
| Streptococcus pyogenes CN10 | 16 |
| S.agalactiae 'Hester' | 16 |
| S.sanguis ATCC 10556 | 16 |
| S.pneumoniae Pu7 | 16 |
| S.faecalis 1 | 4 |

*Multi-resistant (Methicillin, tetracycline, erythromycin and gentamycin resistant)

c) Further purification of MM 55272

The 43mg of solid from the SP Sephadex eluate fractions 18-23 containing MM 55272 was taken up in 0.1M $NaH_2PO_4$ buffer pH 6.0 (25ml) and loaded onto a 22.5ml column containing Matrex silica C18 chromatography medium, equilibrated in the same buffer. The column was eluted with an exponential gradient of 0.1M $NaH_2PO_4$ pH 6.0, to 0.1M $NaH_2PO_4$ pH 6.0 containing 20% acetonitrile (100ml in mixing vessel). 10ml fractions were collected. The fractions were monitored for biological activity and by HPLC. Fractions 21-25 were substantially pure MM 55272. The fractions were bulked and the acetonitrile evaporated off in vacuo. The buffer salts were removed by treatment with D-alanyl-D-alanine affinity resin as previously described to yield 17.4 mg of MM 55272. Fast atom bombardment mass spectrometry indicated a molecular weight of 1473±1.

The antibacterial activity of MM 55272 against a range of Gram positive organisms is shown in the following table.

8

| Antibacterial activity of MM 55272 against a range of organisms, determined by the microtitre method | |
| --- | --- |
| (MIC μg/ml) | |
| Organism | MM 55272 |
| Bacillus subtilis ATCC 6633 | 2 |
| Coynebacterium xerosis NCTC 9755 | 2 |
| Sarcina lutea NCTC 8340 | 4 |
| Staphylococcus aureus | |
| 'Oxford' | 2 |
| 'Russell' | 4 |
| 'V573' MR* | 1 |
| S.saprophyticus 'FLI' | 8 |
| S.epidermidis 60137 | 8 |
| 54815 | 16 |
| Streptococcus pyogenes CN10 | 2 |
| S.agalactiae 'Hester' | 4 |
| S.sanguis ATCC 10556 | 8 |
| S.pneumoniae Pu7 | 4 |
| S.faecalis 1 | 16 |

*Multi-resistant (Methicillin, tetracycline, erythromycin and gentamycin resistant)

Detection Methods

a) Fermentation samples and column fractions were monitored for antibiotic activity by bioassay on Staphylococcus aureus V573, using the conventional hole in plate method.

b) MM 55270, MM 55271 and MM 55272 have a characteristic UV maximum at 280nm. Purified samples can be assayed using direct measurement of this absorbance.

c) Preparations of MM 55270, MM 55271 and MM 55272 were assayed by high performance liquid chromatography using a Waters column (3.9 x 300 mm) containing μ bondapak C18 reverse phase material. [Waters Associates, 34 Maple Street, Milford, Mass., USA]. The glycopeptides were eluted from the column with 0.1M NaH₂PO₄ pH 6.0 containing 11% acetonitrile at a flow rate of 2ml/mm. The eluant was monitored at 220nm by Cecil Instruments CE2112 variable wavelength UV monitor [Cecil Instruments Ltd., Milton Technical Centre, Cambridge, U.K.]. Under these conditions MM 55270 had a retention time of 7.2 minutes and MM 55271 and MM 55272 had identical retention times of 25.0 minutes.

Alternatively the preparations were assayed on a Spherisorb ODS2 reverse phase column, [Phase Separations Ltd., Deeside Industrial Park, Queensferry, Clwyd, U.K]. The glycopeptides were eluted from the column with 0.1M NaH₂PO₄ pH 6.0 containing 12% acetonitrile at a flow rate of 2ml/min. Under these conditions MM 55270 had a retention time of 3.8 minutes and MM 55271 and MM 55272 had identical retention times of 9.0 minutes.

Characterising Data

Physical and spectral properties of MM 55270
FAB-MS (glycerol/thioglycerol/acetic acid)
MH$^+$ 1490±1 Molecular weight 1489±1
HPLC: as above
Physical and spectral properties of MM 55271

FAB-MS (glycerol/thioglycerol/acetic acid)
$MH^+$ 1517±1
Molecular weight 1516±1
HPLC: as above
Physical and spectral properties of MM 55272
FAB-MS (glycerol/thioglycerol/3-nitrobenzyl alcohol)
$MH^+$ 1474±1
Molecular weight 1473±1
Isoelectric focusing pI = ca 8.3
HPLC: as MM55271

## CLASSIFICATION OF NCIB 40086

### Methods used

The methods followed were those recommended by the International Streptomyces Project for the characterization of Streptomyces species [Shirling, E.B. and Gottlieb, D. "Methods for the characterization of Streptomyces species" Int. J. Syst. Bacteriol.,16: 313-340 (1966)] and those recommended for the characterization of Amycolata and Amycolatopsis species [Lechevalier, M.P., Prauser, H., Labeda, D.A. and Ruan, J.-S. "Two new genera of nocardioform actinomycetes: Amyolata gen. nov. and Amycolatopsis gen. nov." Int. J. Syst. Bacteriol. 36: 29-37 (1986)].

The isomers of diaminopimelic acid (DAP) and the carbohydrates in hydrolysates of whole cells were established by Thin Layer Chromatography (TLC) using the methods described by Komagata and Suzuki [Komagata, K. and Suzuki, K.-I. "Lipid and Cell-Wall Analysis in Bacterial Systematics" Methods in Microbiology,19:161-207 (1987)]. Mycolic acids were determined by the methods described by Minnikin et al. [Minnikin, D. E., Hutchinson, I.G. and Caldicott, A.B. "Thin Layer Chromatography of Methanolysates of Mycolic Acid Containing Bacteria" J. Chromatogr. 188: 221-233 (1980)].

### RESULTS.

### 1. Cultural Characteristics:

Culture NCIB 40086 grew well on all the growth media recommended by the ISP, forming well developed, cream to pale brown substrate mycelium and abundant white aerial mycelium. No soluble pigments were detected on any of the media used. The cultural characterisitcs of NCIB 40086 on various media are summarised in Table 1.

### 2. Chemical Characteristics:

Hydrolysed whole-cells of the culture NCIB 40086 contained the meso isomer of diaminopimelic acid. Arabinose and Galactose were the major sugars present while ribose was detected in minor quantities. Mycolic acids were not present. These results indicate that culture NCIB 40086 has a cell-wall type IV with type A sugar pattern (Lechevalier et al, 1986). However, lack of mycolic acids places this culture firmly outside the genus Nocardia sensu stricto.

### 3. Physiological characterisitcs:

Key physiological characterisitcs of culture NCIB 40086 and Amycolatopsis orientalis ATCC 19795, listed in Table 2, show that these two cultures share many characteristics.

### Identification of NCIB 12608:

10

Based on key chemical, physiological and morphological features, culture NCIB 40086 is identified as a strain of Amycolatopsis orientalis. Although NCIB 40086 differs from the type strain of Amycolatopsis orientalis (ATCC 19795) in some tests, the differences are relatively minor and thus, culture NCIB 40086 is identified as a new strain of the species Amycolatopsis orientalis.

Table 1:

| Growth characteristics of NCIB 40086 after 14 days at 28° C | | | | | |
|---|---|---|---|---|---|
| MEDIUM | GROWTH | AERIAL MYCELIUM | | SUBSTRATE MYCELIUM | SOLUBLE PIGMENT |
| | | Formation | Colour | | |
| ISP 2 | Good | Good | White | Cream | None |
| ISP 3 | Good | Fair | White/cream | White/cream | None |
| ISP 4 | Good | Good | White | Cream | None |
| ISP 5 | Good | Good | White | Cream | None |
| ISP 6 | Fair | Poor | White | White/cream | None |
| ISP 7 | Good | Good | White | Cream/white | None |
| Nutrient Agar | Good | Fair | White | Cream | None |
| Bennetts Agar | Good | Good | White | Cream | None |
| Czapek-Dox Agar | Good | Fair | White | Cream/White | None |

Table 2:

Physiological characterisitics of NCIB 40086 after 14 days at 28°C

| CHARACTERISTICS | NCIB 40086 | Amycolatopsis orientalis ATCC 19795 |
|---|---|---|
| **Decomposition of:** | | |
| Adenine | – | – |
| Casein | + | + |
| Hypoxanthine | – | + |
| Tyrosine | + | + |
| Xanthine | + | + |
| **Production of:** | | |
| Nitrate reductase | + | + |
| Amylase | + | + |
| Urease | + | + |
| Melanin | – | – |
| Esculinase | + | + |
| Gelatinase | ND | + |
| **Decarboxylation of:** | | |
| Benzoate | – | – |
| Citrate | – | + |
| Mucate | – | – |
| Malate | – | + |
| **Growth in the presence of:** | | |
| Lysozyme (500 u./ml) | + | – |
| Salicylate | + | – |
| NaCl (5%, w/v) | + | + |
| Rifampicin (50 ug/ml) | + | + |
| **Growth at:** | | |
| 28°C | + | + |
| 37°C | + | – |
| 45°C | – | – |
| **Utilization of carbohydrates as sole carbon sources:** | | |
| Adonitol | – | + |
| Arabinose | – | + |
| Dextrin | – | + |
| Erythritol | – | + |
| Galactose | + | + |
| Glucose | + | + |
| Inositol | +/– | + |
| Lactose | + | + |
| Maltose | – | + |
| Mannitol | + | + |
| Mannose | + | + |
| Melibiose | + | + |
| ⍺-methyl-D-glucoside | + | + |
| Raffinose | + | – |
| Rhamnose | – | + |
| Salicin | +/– | + |
| Sorbitol | – | – |
| Sucrose | + | + |
| Trehalose | + | + |
| Xylose | + | + |
| (Control : no sugar) | – | – |

Key:   +/– = variable result;   ND = Not determined

12

**Claims**

1. A complex of glycopeptide compounds, or the compound MM55270, MM55271, or MM55272, as hereinbefore defined.

2. A complex or compound according to claim 1, in substantially pure form.

3. A process for the production of a complex or compound according to claim 1, which comprises cultivating a producing microorganism and subsequently isolating the complex or compound or a derivative thereof from the culture.

4. A process according to claim 3, wherein the microorganism is Amycolatopsis sp. NCIB 40086 or a mutant thereof.

5. Amycolatopsis sp. NCIB 40086 or a mutant thereof, in biologically pure form.

6. A pharmaceutical composition comprising an antibacterially effective amount of a complex or compound according to claim 1, or a pharmaceutically acceptable derivative thereof, together with a pharmaceutically acceptable carrier or excipient.

7. A complex or compound according to claim 1, or a pharmaceutically acceptable derivative thereof, for use in therapy.

8. A complex or compound according to claim 1, or a pharmaceutically acceptable derivative thereof, for use in the treatment of bacterial infections.

9. Use of a complex or compound according to claim 1, or a pharmaceutically acceptable derivative thereof, in the manufacture of a medicament for use in the treatment of bacterial infections.

10. A method of treating bacterial infections in animals, which comprises administering an antibacterially effective amount of a complex or compound according to claim 1, or a pharmaceutically acceptable derivative thereof, to an animal in need thereof.

Claims for the following Contracting State: ES

1. A process for the production of a complex of glycopeptide compounds, or the compound MM55270, MM55271, or MM55272, as hereinbefore defined, which comprises cultivating a producing microorganism and subsequently isolating the complex or compound or a derivative thereof from the culture.

2. A process according to claim 1, wherein the complex or compound is produced in substantially pure form.

3. A process according to claim 1 or 2, wherein the microorganism is Amycolatopsis sp. NCIB 40086 or a mutant thereof.

4. Amycolatopsis sp. NCIB 40086 or a mutant thereof, in biologically pure form.

5. A process for the preparation of a pharmaceutical composition, comprising admixing an antibacterially effective amount of a complex or compound as hereinbefore defined, or a pharmaceutically acceptable derivative thereof, with a pharmaceutically acceptable carrier or excipient.

6. A method of treating bacterial infections in animals, which comprises administering an antibacterially effective amount of a complex or compound as hereinbefore defined, or a pharmaceutically acceptable derivative thereof, to an animal in need thereof.

13

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | JOURNAL OF THE AMERICAN CHEMICAL SOCIETY, vol. 110, 1988, pages 7896-7897, American Chemical Society; R. NAGARAJAN et al.: "M43 antibiotics Methylated vancomycins and unrearranged CDP-I analogues" * Complete article * --- | 1-6 | C 12 P    1/00 C 12 P   21/00 A 61 K   37/00 C 07 G   11/00 // (C 12 P    1/00 C 12 R    1:01 ) |
| Y | THE JOURNAL OF ANTIBIOTICS, vol. 41, 1988, pages 1506-1510; N. TSUJI et al.: "New glycopeptide antibiotics: II. The isolation and structures of chloroorienticins" * Complete article * --- | 1-6 | |
| Y | THE JOURNAL OF ANTIBIOTICS, vol. 41, no. 11, 1988, pages 1525-1532; K. DOBASHI et al.: "Novel antifungal antibiotics octacosamicins A and B I. Taxonomy, fermentation and isolation, physico-chemical properties and biological activities" * Complete Article * --- | 1-6 | **TECHNICAL FIELDS SEARCHED (Int. Cl.5)** |
| P,Y | THE JOURNAL OF ANTIBIOTICS, vol. 42, no. 10, 1989, pages 1453-1459; M.S. PACEY: "UK-69,753, A novel member of the efrotomycin family of antibiotics I. Taxonomy of the producing organism, fermentation and isolation" * Complete article * --- -/- | 1-6 | C 12 P C 12 R A 61 K |

The present search report has been drawn up for all claims

| Place of search | Date of completion of the search | Examiner |
|---|---|---|
| THE HAGUE | 20-03-1990 | RAJIC M. |

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A | CHEMICAL ABSTRACTS, vol. 111, 1989, page 314, abstract no. 92629y, Columbus, Ohio, US; M.J. ZMIJEWSKI Jr. et al.: "Biosynthesis of vancomycin: identification of TDP-glucose:aglycosyl-vancomycin glucosyltransferase from amycolatopsis orientalis", & FEMS MICROBIOL. LETT. 1989, 59(1-2), 129-33 * Abstract * | 1 | |
| P,Y | EP-A-0 309 161 (BEECHAM) * Completely * | 1-6 | |

TECHNICAL FIELDS SEARCHED (Int. Cl.5)

The present search report has been drawn up for all claims

| Place of search | Date of completion of the search | Examiner |
|---|---|---|
| THE HAGUE | 20-03-1990 | RAJIC M. |

EPO FORM 1503 03.82 (P0401)